# EUROPEAN PATENT APPLICATION

(11) **EP 4 487 840 A1**
(43) Date of publication of application: **08.01.2025**
(21) Application number: 23184215.4
(22) Date of filing: 07.07.2023
(51) Int. Cl.: A61K 9/70, A61K 31/565, A61K 31/57, A61K 47/32

(54) **A THREE-LAYERED INTRAVAGINAL RING**

(71) Applicant: Sever Pharma Solutions, 212 15 Malmö (SE)
(72) Inventor: De Graaff, Wouter, 212 15 Malmö (SE); Refaa, Zakariaa, 212 15 Malmö (SE)
(74) Representative: Holme Patent A/S

(57) **Abstract**

The present invention relates to a three-layered intravaginal ring (1;1'), a core (2;9), an intermediate layer (3;10), and a sheath (6) comprising a second ethylene-vinyl acetate copolymer (7) having a vinyl acetate content from 20 to 40 wt%, and at least 10 wt% of a progestational steroid (8) based on the weight of the sheath, wherein one of the core (9) and intermediate layer (3) comprises a first ethylene-vinyl acetate copolymer (4) having a vinyl acetate content from 26 to 40 wt% and an estrogenic steroid (5), and the other one of the core (2) and intermediate layer (10) is an inactive layer (2;10). Using the intravaginal ring according to the present the inventors have found that it is possible to attain independent and optimal release of the two active ingredients; an estrogenic steroid and a progestational steroid, without the need for complex assembly of parts.

## Description

The present invention relates to a three-layered intravaginal ring, and a method of manufacturing said ring.

Various types of intravaginal rings (IVRs) have been developed for the controlled and sustained release of active ingredients preferably by diffusion through the surface of the ring.

Examples of such devices are the Estring^{®}, Femring^{®}, and Nuvaring^{®}, all of which provide controlled and sustained release of steroid molecules over a prolonged period, e.g. several weeks/months. In addition to preventing undesired pregnancies, the device provides several advantages: its use is controlled by the female; it allows for a better regulated dose of drug without attention by the user; and it avoids the destruction (by the intestine and by first pass through the liver) of an appreciable portion of the daily dosage of the drugs compared to their orally delivered counterparts.

These known vaginal rings have been found particularly useful for the release of steroids, whose relatively small molecular size and substantially water-insoluble nature permit effective permeation through the hydrophobic elastomer/polymer, such that therapeutic concentrations may be readily achieved in the body.

However, diffusion in polymers is complex and is known to depend on a number of different factors, e.g. temperature, the manufacturing process, the solubility and diffusivity of the drug in the polymer, the surface area of the drug reservoir, the distance the drug must diffuse through the device to reach its surface and the molecular weight of the drug. Consequently, it remains a challenge to understand, predict and control the diffusion of small and large molecules in polymer systems. In this respect, the use of intravaginal rings to deliver drugs requires a design that regulates the release rate so as to reliably provide the user with the appropriate daily dose throughout the lifetime of the device.

In reservoir systems, i.e. a drug loaded core surrounded by a non-medicated membrane/sheath, the drug first partitions into the sheath from the reservoir and then diffuses to the other side of the sheath, where it is taken up by the receiving medium. While the reservoir is saturated, a constant concentration gradient of drug is maintained in the membrane, the rate of drug flux is constant, and zero order release is achieved. However, when drug concentration in the reservoir falls, the gradient across the membrane and the release rate of the drug also decreases.

Furthermore, reservoir systems can be difficult to fabricate reliably, and pinhole defects and cracks in the sheath surrounding the reservoir, can lead to dose dumping, i.e. unintended, rapid drug release over a short period of time.

Simultaneous drug delivery/release finds application in a number of different areas. However, the placement of a blend of drugs in a single intravaginal ring in a proportion equal to the desired delivery rate ratio will almost never achieve the desired result. In many cases, the drugs will not diffuse together through the surface or membrane at the same ratio, as they exist in the blend. The ratio would instead be dependent on the inherent ratio of the normalized permeation rates for the drugs through e.g. a rate-controlling membrane. Flexibility would therefore be limited to the selection of suitable polymer candidates for the sheath. Accordingly, the range of, and degree of control over, the delivery rate ratio, is extremely limited.

Of course the need for maintaining a specified delivery rate ratio can be met by using a separate intravaginal ring for each drug. However, this is clearly undesirable, since the presence of two or more intravaginal rings will compound the disruption which even a single ring might create in the normal physiological activity of an animal or human. In addition, if one intravaginal ring malfunctions, the desired delivery ratio will be lost. Further, complete therapy in a single intravaginal ring is more acceptable to patients and more efficient to insert and remove.

Adjustment of a specified delivery rate can also be met by two or multi-compartments intravaginal rings, and ring bodies containing drug release capsules. The industrial scale manufacturing of such rings is however complex and expensive.

A further problem with the known intravaginal rings arranged for releasing more than one drug is that such rings usually show sub-optimum release patterns for the different drugs, whereas it is generally preferred that all drugs are released in a controlled rate during a specified duration of time.

Thus, there is a demand for a novel intravaginal ring arranged for releasing two active ingredients/drugs in a controlled manner and in the correct ratio, and a method for manufacturing the system that is simple and inexpensive.

Thus, it is a first aspect of the present invention to provide a three-layered intravaginal ring, which can be loaded with both an estrogenic steroid and a progestational steroid and wherein each steroid is released at a controlled rate independently of the other steroid.

In a second aspect according to the present invention is provided a three-layered intravaginal ring that reduces the variability of the release rate of steroids over time.

In a third aspect according to the present invention is provided a three-layered intravaginal ring in which the known problems relating to complicated and expensive manufacturing processes, dose dumping and initial drug burst are eliminated, and which at the same time provides a substantially zero-order release rate of the estrogenic steroid.

In a fourth aspect according to the present invention a provided a three-layered intravaginal ring which can comprise a relatively low concentration of estrogenic steroid in solid form and still maintain a desired release rate of said steroid.

The novel and unique features whereby these and further aspects are achieved according to the present invention is by providing a three-layered intravaginal ring comprising
- a core,
- an intermediate layer, and
- a sheath comprising a second ethylene-vinyl acetate copolymer having a vinyl acetate content from 20 to 40 wt%, and at least 10 wt% of an progestational steroid based on the weight of the sheath,
wherein one of the core and intermediate layer comprises a first ethylene-vinyl acetate copolymer having a vinyl acetate content from 26 to 40 wt%, and an estrogenic steroid, and the other one of said core and intermediate layer is a inactive layer.

The intravaginal ring according to the invention relates to a system that comprises a core surrounded, at least partly and preferably completely, by first an intermediate layer (shell) and then completely by a sheath, which is the outer layer of the vaginal ring. The three-layers of the intravaginal ring are preferably co-axially/concentric arranged, i.e. the core, intermediate layer and sheath share a common axis.

Such a three-layered intravaginal ring provide an improved delivery system compared to the known delivery systems, since the release rate of the estrogenic steroid and the progestational steroid can be adjusted independently from one another.

The estrogenic steroid may be present in either the core or the intermediate layer, but not in both the core and intermediate layer, at least not initially, e.g. at the time of manufacturing. Thus, in a first embodiment of the intravaginal ring according to the invention the intermediate layer comprises the estrogenic steroid and the core is the inactive layer; and in a second embodiment the core comprises the estrogenic steroid and the intermediate layer is the inactive layer. The sheath is the same for both embodiments.

Within the context of the present invention the term "inactive layer" means a layer/section of the vaginal ring, which in the present case may be either the core or intermediate layer, that has no active properties, i.e. said inactive layer does not contain or comprise any active ingredients such as medicaments, steroids, or other components that may have an active effect on the patient and/or the intravaginal ring. A person skilled in the art will understand that a small part of the steroids present in the three-layered vaginal ring will dissolve in the ring, and that a fraction of the dissolved steroid may/will re-distribute in the vaginal ring until a thermodynamic equilibration has been reached. Thus, the point of time when the core and/or intermediate layer are the inactive layer, i.e. has no active properties, is before said equilibration process has been initiated, e.g. at the time of manufacture. The equilibration process may take several weeks to months depending on the dimensions of the core, intermediate layer and sheath, as well as on storage and use conditions.

According to the present invention the estrogenic steroid is placed in either the core or intermediate layer, but contrary to the conventional vaginal rings where the sheath comprises no active ingredient e.g. known from WO2009/036999 and WO2004/103336, the sheath of the present invention comprises a high concentration of progestational steroid (at least 10wt%), whereby simultaneous and individual release of both the progestational steroid and the estrogenic steroid is provided.

It must be noted that even though it is known to have small concentrations of active ingredient in a sheath surrounding a drug loaded core, see e.g. WO2013/120888 and WO2015/086489, it is not known to have a progestational steroid in the sheath (as the outer layer) in the high concentrations claimed in the present invention. Small concentrations of progestational steroid i.e. concentrations well below 10 wt%, will have no or only a very limited effect on the release rate of the estrogenic steroid in the core/intermediate layer, and are accordingly not relevant for the present invention.

Dual administration of both a progestational steroid and an estrogenic steroid finds application in a number of different areas, e.g. in contraceptive vaginal rings and in vaginal rings providing hormone replacement. For such intravaginal rings it is required to release the two steroids simultaneously and at the same time. It is therefore necessary to adjust the release rate of these steroids independently to the physiological optimal rate (mg/day). Using the intravaginal ring according to the present invention the inventors have found that it is possible to attain independent and optimal release of the two steroids; an estrogenic steroid and a progestational steroid without the need for complex assembly of parts.

Thus, using an intravaginal ring in which the concentration of the progestational steroid in the sheath is at least 10wt%, it is ensured that the desired near zero-order release behavior of the estrogenic steroid in either the intermediate layer or in the core are observed for a longer period of time. In addition such high concentrations of the progestational steroid are associated with good physical stability of said steroid.

By substantially zero order is meant that a substantially constant amount of the estrogenic steroid is released over a given period of time. In some embodiments, the system exhibit a substantially zero order release profile of the estrogenic steroid over a treatment period of at least 14 days, preferably at least 28 days, and even more preferred around two to three months.

As discussed earlier it is a well known problem that if the core contains an active ingredient, the release rate of said active ingredient decreases as active ingredient(s) that is deeper inside the core/reservoir must diffuse to the surface.

This problem is eliminated by providing a vaginal ring comprising an inactive layer in the form of either an inactive core or an inactive intermediate layer. Thus, having a three-layered vaginal ring comprising a inactive layer (core or intermediate layer), an opposite core or intermediate layer comprising the estrogenic steroid, and a sheath comprising a high concentration of a progestational steroid, the inventors of the present invention has found that the release rate of the estrogenic steroid from the core or the intermediate layer, is substantially constant over time, thereby providing a substantially zero-order release rate of the estrogenic steroid during the desired treatment period. For the embodiment with the inactive core the zero order release rate can be attributed to the barrier properties of the sheath, where the particles of the progestational steroid will act as 'filler' and likely contribute to the barrier properties of said sheath. For the embodiment with the inactive intermediate layer, zero order release is likely attributed to the combined barrier properties of ann inactive intermediate layer and the sheath with particles of progestational steroid.

The three-layered intravaginal ring according to the invention may have the estrogenic steroid present in either the core or the intermediate layer, the other being the inactive layer, and even though both embodiments/constructions provide an improved release rate and lower steroid burst, the two different ring embodiments also have individual advantages.

For instance, when the estrogenic steroid is present in the intermediate layer and the core is the inactive layer (inactive core), the inventors of the present invention have found that the use of a inactive core (layer) ensures that a small fraction of the estrogenic steroid loaded in the intermediate layer and a small fraction of the progestational steroid loaded in the sheath can diffuse back into the "empty" inactive core. This mechanism can/will eliminate any persisting super saturation and provide a more reliable and constant release rate, and a lower initial burst, of said steroids. Thus, said embodiments provide an intravaginal ring with an even better release profile than hitherto known.

Furthermore, desirable release rates of the estrogenic steroid can be obtained with lower amounts of estrogenic steroid placed in the intermediate layer of the three-layered ring according to the invention, compared to a two-layered intravaginal ring (core/sheath) in which the estrogenic steroid is present in the core. For instance, if a two-layered intravaginal ring comprises 10wt% of the estrogenic steroid an acceptable physical stability of the ring is obtained, however this will also result in an excess drug load. Loading with an even higher concentration of estrogenic steroid in the core of a two-layered ring would result in a further increased excess of estrogenic steroid and other unfavorable properties like too high stiffness of the ring.

In a three-layer design however the volume of the intermediate layer can be controlled and reduced or increased if required. Hence much higher drug loads can be used without overloading the system. Using e.g. 25wt% of estrogenic steroid in the intermediate layer would have the advantage of flatter profiles as at the same release rate the penetration of the depleted layer is less deep.

The inventors of the present invention have in this respect compared a two-layered intravaginal ring (core/sheath) with a three-layered intravaginal ring according to the invention (estrogenic steroid placed in the intermediate layer). Thus, in the two-layered ring estradiol (estrogenic steroid) is present in the core, and in three-layered ring estradiol is present in the intermediate layer, the core being the inactive layer. The ethylene-vinyl acetate copolymers were the same for the layers containing the respective steroids. Both rings were surrounded by a sheath with about 33,9 wt% progesterone (progestational steroid.) The inventors found that a release rates around 160µg/day could be obtained with the three-layered ring using significantly less of the estradiol concentration required for obtaining the same release rate. In one example, the inventors found that for a release rate of estradiol of about 250 µg/day, the two-layered ring required 210-215 mg estradiol in order to obtain said release rate of about 28 days, and the three-layered ring (5 wt% estradiol in a 200 µm thick intermediate layer) only required about 17-18 mg estradiol.

It should in this respect be noted that in a two-layered intravaginal ring higher drug loads aims at provides a higher degree of physical stability in the intravaginal ring. However, the inventors of the present invention found that only a small percentage of said drug amount were in fact used for therapeutic purposes. Although the two-layered ring provided a zero order release, and was physically stable, the use of a three-layered intravaginal ring according to the invention, with an inactive core, and the estrogenic steroid placed in an intermediate layer, is a clear improvement over the two-layered ring. Said improvement relates to both the benefit of being able to tailor the amount of active ingredients loaded in the intravaginal ring, and to the fact that a higher percentage of the estrogenic steroid loaded in the intravaginal ring can be delivered to the surrounding environment, leaving a reduced remnant content of estrogenic steroid in the intravaginal ring after use, thereby both reducing manufacturing cost and the impact on the environment.

Furthermore, since the thickness (volume) of the intermediate layer comprising the estrogenic steroid can be adjusted, the drug load of the estrogenic steroid in the three-layered vaginal ring according to the invention can be tailored to meet specific demands, e.g. a certain drug load to sustain the release of said steroid for the intended duration of use.

In relation to the ring-construction in which estrogenic steroid is present in the core, and the intermediate layer is the inactive layer, said embodiment has the surprising advantage than the intermediate inactive layer will act as a rate limiting barrier layer, that can be used to adjust the release profile of the estrogenic steroid in the core, without affecting progestational steroid release from the sheath. In this way it is possible to attain independent and optimal release of the two steroids in a simpler way than hitherto known.

For comparison purposes, it should be noted that for a two-layered intravaginal ring (core/sheath) wherein the estrogenic steroid is present in crystalline state in the core and surrounded by a sheath, the concentration of estrogenic steroid driving release is equal to the saturation concentration. Hence, varying the estrogenic steroid drug load will not substantially affect release of the estrogenic steroid when said steroid is present in crystalline state. Thus, the only way to influence estrogenic steroid release in a two-layered ring is by varying the permeation resistance (barrier properties) of the progestational steroid sheath. The barrier properties of the sheath are determined by sheath thickness, the properties of the sheath polymer and the content of progestational steroid particles in the sheath.

However, the properties of the polymer and the content of progestational steroid particles cannot be changed without affecting progestational steroid release and hence the only variable left to adjust the estrogenic steroid release independently of other factors, is the sheath thickness.

In this respect the inventors found, based on simulations and experimental data, that a sheath having a thickness of at least 400µm, such as 500 - 600 µm is required to reduce estrogenic steroid release to one desired range of about 160µg/day in a two-layered ring comprising about 10 wt% estradiol in the core and about 33.9wt% progesterone in the sheath. If a lower estrogenic steroid release was desired an even thicker sheath would be required. The latter is hampered by fundamental physics resulting in practical limitations.

The estrogenic steroid loaded in solid state (particles) in the core will be in equilibrium with the steroid dissolved in the core at saturation solubility. This small fraction of dissolved steroid will re-distribute in the vaginal ring until uniform activity through-out the intravaginal ring (thermodynamic meaning) has resulted. For practical reasons, it is required that this equilibration process is relatively rapid as only after equilibration stable release profiles are obtained. Furthermore, as is confirmed by the data in the present invention a reduction to 80µg/day or lower would require sheath thicknesses above 700 µm, which would lead to unacceptable equilibration times. Accordingly, such high sheath thicknesses are not desired from a practical point of view.

However, the inventors of the present invention have found that by a three-layered ring, in which the estrogenic steroid is placed in the core, and an inactive intermediate layer is placed between the sheath and the core, will solve the above problems, as the inactive intermediate layer will function as additional barrier layer whereby the release rate of the estrogenic steroid can be varied without affecting progestational steroid release from the sheath. For instance if a lower release rate of the estrogenic steroid is desired, the inactive intermediate layer can be thicker and vice versa. Alternatively, the inactive intermediate layer can be made of e.g. an ethylene-vinyl acetate copolymer with a lower or higher VA-content in order to adjust the release rate to the desired level.

Thus, the use of a three-layered ring according to the present invention in which the intermediate layer is the inactive layer provides an optimal intravaginal ring compared to a corresponding two-layered vaginal ring, i.e. without the intermediate inactive layer.

In order to obtain one or more of the above advantages, it is relevant that the progestational steroid is dispersed and/or incorporated in the second ethylene-vinyl acetate copolymer to an extent sufficient to control the diffusion rate of the estrogenic steroid through the sheath.

Without being bound by theory, it is believed that the progestational steroid in the sheath will function as a filler and control the release rate of the estrogenic steroid, irrespectively of whether the estrogenic steroid is present in the core or in the intermediate layer.

When the progestational steroid present in the sheath is released to the surroundings, the concentration of said progestational steroid is reduced and it is believed that the diffusion of water into the sheath may be facilitated leaving behind an empty porous matrix and/or empty pockets/holes and/or the sheath may collapse thereby ensuring the desired zero-order release profile of the estrogenic steroid. Thus, it is believed that the space initially occupied by the progestational steroid leaves behind an empty porous matrix which may become water filled due to ingress of water and/or may leave behind empty pockets/holes. This is contrary to the conventional findings in which the release rate of an active ingredient in a core surrounded by a non-medicated sheath slightly decreases over time, i.e. the desired zero order release rate cannot be maintained over a desired treatment period for such conventional vaginal rings.

In some embodiments it is preferred that at least 15 wt% of the progestational steroid is dispersed in the second ethylene-vinyl acetate copolymer, even more preferred at least 20 wt%, and even more preferred at least 25 wt%.

In order to ensure that the estrogenic steroid can be released through the sheath it is preferred that the sheath comprises a concentration of the progestational steroid based on the weight of the sheath of not more than 40 wt%, preferably 35 wt% or below.

The presence of the progestational steroid in the relatively high concentrations (e.g. between 10 and 40 wt%) in the sheath will not only lead to an increase in the mean path length the molecules of the estrogenic steroid have to travel between two points in the sheath, but will also reduce the amount of the estrogenic steroid which can be dissolved in the second ethylene-vinyl acetate copolymer of the sheath, accordingly decreasing the release rate of the estrogenic steroid though said sheath. Accordingly, the sheath can be made smaller, providing a smaller product with a significant lower burst of the estrogenic steroid.

It is in this respect preferred that the progestational steroid is incorporated and/or dispersed in the second ethylene-vinyl acetate copolymer in the form of particles, preferably crystals. Such particles/crystals will form a repository of solid, undissolved crystals which will act as seed crystals i.e. as a slow release depot. Over time, when the progestational steroid is delivered to the surroundings, some of the crystals will be unlocked in the second ethylene-vinyl acetate copolymer, thereby providing a prolonged release of the progestational steroid. Furthermore, the stability of the progestational steroid in the intravaginal ring is improved when the progestational steroid is incorporated into the sheath as undissolved particles/crystals.

Without being bound by theory it is believed that maintaining a concentration of the progestational steroid at high concentrations in the sheath it is assumed that a porous network path is created by crystals and wherein a number of sites/openings/pores in the matrix of the second ethylene-vinyl acetate copolymer remains empty, ensuring that the estrogenic steroid only can be released through a tortuous path in the sheath, thereby the diffusion length increases and the release rate is controlled.

A person skilled in the art will based on the context of the present invention understand that it will be possible to control and/or adjust the diffusion rate through the sheath by varying the amount/concentration of the progestational steroid in said sheath, by using different grades of the second ethylene-vinyl acetate copolymer, and/or by using different particle sizes of the progestational steroid or blends of different particles sizes.

In order to obtained the desired zero order release profile of the estrogenic steroid during the treatment period, it is preferred that the estrogenic steroid is present in the intermediate layer or core in the form of particles, preferably crystals, for the same reasons as disclosed for the progestational steroid. In this way the stability of both the estrogenic steroid and progestational steroid, and accordingly the intravaginal ring according to the intention, is improved.

When the estrogenic steroid is present as particles in either the core or intermediate layer, it is preferred that said estrogenic steroid is dispersed and/or incorporated in the first ethylene-vinyl acetate copolymer of the core or intermediate layer in a concentration of at least 5 wt% of the total weight of the respective intermediate layer or core, preferably at least 10 wt%, and even more preferred at least 15 wt% or even higher e.g. at least 20 wt%. A load of 5wt% is above estradiol (estrogenic steroid) saturation concentration, but such high loads is preferred to avoid delayed crystallization of estradiol which may dissolve in the polymer e.g. during the extrusion process.

Due to a low solubility of the steroids in the first and second ethylene-vinyl acetate copolymer, the use of the relatively high drug loads used (at least 10wt% progestational steroid and at least 5 wt% estrogenic steroid) assist with ensuring that said steroids will be incorporated in the first and second ethylene-vinyl acetate copolymer as particles, preferably in a crystalline state.

In a preferred embodiment the particles/crystals of the progestational steroid and the estrogenic steroid have a mean particle size of between 1 µm and 40 µm, preferably between 2 µm and 24 µm, and even more preferred between 3 and 10 µm, such as around 5µm as it has been proven that such particle sizes provide the desired near zero order relate rate for a prolongs period of time, i.e. for at desired treatment period of at least 14 days, preferably at least 28 days e.g. one to two months..

As used herein, the term "crystals" refers to particles of the steroids arranged in an ordered microscopic structure, forming a crystal lattice. The term "crystal size" or "particle size" refers to a crystal's or particle's mean particle diameter. Particle size, crystal size and/or particle size distribution is preferably measured using laser diffraction e.g. using a Malvern laser scattering particle size analyzer. However any other particle size measurement apparatus or technique known to person's skilled in the art can also be used, e.g. dynamic light scattering, a sieve analysis or a microscopic determination. As used herein, the term "crystal size" or "particle size" relates to the particle distribution diameter of the particle/crystal. As an example can be mentioned that D50 means that 50% of the particles have a diameter below the given value when measured using e.g. laser diffraction, dynamic light scattering, or a sieve analysis. For instance, for the steroid progesterone the D50 is preferably less than 10 µm, and the particle size is usually between 4 - 6 µm, such as around 5 µm. For the steroid estradiol, D90 is less then 5 µm.

The first and second ethylene-vinyl acetate (EVA) copolymers, used in the intravaginal ring of the present invention are suitable for placement in the vaginal tract, i.e. the materials are non-toxic and non-absorbable in a patient or an animal, and are considered to have both excellent mechanical and physical properties.

The vinyl acetate concentration of the EVA-copolymer determines the rate of diffusion of the active ingredient through the system and generally, the lower the vinyl acetate concentration, the slower the active ingredient will be release from the copolymer or migrate through it.

In order to provide the desired zero order release profile of the estrogenic steroid it is preferred that the first ethylene-vinyl acetate copolymer comprises an ethylene-vinyl acetate copolymer with a vinyl acetate content from 26 to 40%, preferably 26%, 33% or 40%.

In a similar way the second ethylene-vinyl acetate copolymer preferably has a vinyl acetate content from 20 to 40 wt%, such as e.g. 24 wt%, 28 wt%, 33 wt% or 40 wt% as these materials will provide the desired release profile through the sheath, e.g. by ensuring that the progestational steroid release rate is at the optimal level.

Since the polymers used for the inactive core will not directly affect release of the steroids in the sheath and intermediate layer, the polymers of the inactive layer may in principal be made of any relevant polymer that are suitable for placement in the vaginal tract.

However, when the inactive layer is the core, it is preferred that said inactive core comprises a third ethylene-vinyl acetate (EVA) copolymer e.g. having a vinyl acetate content from 1 to 33 wt%, or alternatively a thermoplastic polyurethane (TPU) or a low-density polyethylene (LDPE).

LowVA-content EVAs, typically between 9 and 20wt% vinyl acetate, is especially relevant if a reduced back diffusion into the inactive core is desired, and EVA with a VA-content of 33 wt% may be useful in case a sink for persisting super saturation is needed as it can contain more dissolved steroid.

TPU or LDPE is relevant for three-layered vaginal rings with a smaller cross-sectional diameter, e.g. around 3 - 4 mm is desired in order to maintain sufficient ring stiffness for said vaginal ring to resist compression forces when placed in the vaginal tract. In this respect the relevant stiffness may also be achieved by using polymers with relatively high tensile modulus or by increasing cross-sectional diameter or by adjusting both to arrive at the desired target.

These materials will provide the desired mechanical strength of the three-layered vaginal ring and at the same time ensure that the core as an inactive layer can function as an empty reservoir allowing the steroids from the intermediate layer and sheath to diffuse into said core in order to eliminate any persisting super saturation in said intermediate layer and sheath, thereby providing a vaginal ring with constant release profiles and lower burst release. The inventors of the present invention have found that a three-layered vaginal ring with a inactive core, relative to a intravaginal ring having a core loaded with solid particles improves the mechanical properties of the vaginal ring, e.g. provides a reduced stiffness, and thereby better flexibility and recovery of shape after compression.

When the inactive layer is the intermediate layer, it is preferred that said inactive intermediate layer is made of a linear polyethylene (LPE) or an ethylene-vinyl acetate (EVA) copolymer having a VA content above 0 wt% but not more than 24 wt%, preferably 6wt% - 20wt%, even more preferably 9wt% - 18wt%, e.g. 15wt% (EVA15) as these polymers have proven highly efficient in acting as a barrier layer to adjust the release profile of the estrogenic steroid in the core, without affecting progestational steroid release from the sheath.

In one embodiment of the present invention the first and second and optionally the third ethylene-vinyl acetate copolymer have the same vinyl acetate content, i.e. the first and second and optionally the third ethylene-vinyl acetate copolymer are the same.

When a specific vinyl acetate (VA) content e.g. 20 wt% is mentioned it refers to the weight% content provided by the manufacture. However, manufactures may use different internal analytical methods for determining vinyl acetate content, and there may therefore be variations in the range of 1 - 2 % in the actual vinyl acetate content depending on the manufacture. Thus, in the present invention the vinyl acetate content refers to the vinyl acetate content in the ethylene-vinyl acetate copolymer determined by high resolution NMR according to standard methods. The wt% of the vinyl acetate content in the ethylene-vinyl acetate copolymer is the wt% content based on the weight of the ethylene-vinyl acetate copolymer.

The intravaginal ring according to the invention is a dual-drug delivery intravaginal ring i.e. it comprises both an estrogenic steroid and a progestational steroid. In a preferred embodiment the estrogenic steroid is estradiol or estriol and/or the progestational steroid is selected from a group consisting of a progestogen, progesterone, etonogestrel, levonorgestrel, d-1-norestrel, segesterone and norethindrone, preferably levonorgestrel. The steroids can be selected for preventing contraception, or to treat a conditions, e.g. vaginal atrophy, or for hormone replacement therapy e.g. relating to symptoms associated with menopause, such as hot flashes.

The intravaginal ring according to the invention is adapted to deliver pharmaceutically effective amounts of the steroids. By "pharmaceutically effective," it is meant an amount, which is sufficient to affect the desired physiological or pharmacological change in the subject. This amount will vary depending upon such factors as the potency of the steroid, the desired physiological or pharmacological effect, and the time span of the intended treatment. Those skilled in the arts will be able to determine the pharmaceutically effective amount for any given steroid in accordance with standard procedures.

The thickness of the sheath, which is the outer layer of the three-layered intravaginal ring according to the present invention, can be varied to further control the release rate of the estrogenic steroid.

The dimensions of the intravaginal ring may vary depending upon the anatomy of the subject, the amount of active ingredient to be delivered to the patient, the time over which the active ingredient is to be delivered, the diffusion characteristics of the active ingredient and other manufacturing considerations. The only requirement being that the intravaginal ring should be flexible enough to enable bending and insertion inside the vaginal cavity and rigid enough to withstand the expulsive forces of the vaginal musculature without causing abrasion to the vaginal epithelium. The outer diameter of such intravaginal rings may range, e.g., from about 45 mm to about 65 mm, and/or the length of the fiber elements forming the intravaginal ring may have a length from 150 to 170 mm, preferably from 154 to 160 mm, such as about 157 mm. The surface area of the intravaginal ring determines the drug release, and hence larger diameter rings are assumed to display proportionally higher drug release.

In a first embodiment, wherein the core is the inactive layer, i.e. the intermediate layer comprises the estrogenic steroid, said intermediate layer preferably has a thickness from 50 µm to 500 µm, preferably from 80 µm to 400 µm, and even more preferred from 100 µm to 300 µm, as such a thicknesses have proven to provide the desired release rate of the estrogenic steroid from the intermediate layer. In certain embodiments the thickness of the intermediate layer is 100 µm, 200 µm, or 300 µm.

In an embodiment wherein the intermediate layer is the inactive layer, i.e. it is the core that comprises the estrogenic steroid, said intermediate layer preferably has a thickness from 50 µm to 300 µm, preferably from 50 µm to 200 µm, and even more preferred from 80 µm to 200 µm, as such thicknesses will provide the desired rate controlling barrier. In certain embodiments the thickness of the intermediate layer is 80 µm, 100 µm, or 200 µm.

The core preferably has a round cross-section with a cross-sectional diameter of between 2 and 8 mm, more preferably between 3 mm and 6 mm and even more preferably from 4 to 5 mm. Said core diameter may apply to both constructions, i.e. irrespectively of whether the inactive layer is the core or the intermediate layer.

The thickness of the sheath determines the amount of progestational steroid present in the ring, assuming the steroid concentration is uniform/not varied. The amount of progestational steroid loaded in the intravaginal ring is an important design parameter as the progesterone release should be sustained over the intended duration of use and on the other hand a large excess should be avoided for economic and environmental reasons. In a preferred embodiment, the thickness of the sheath (for both embodiments) may be between 0.05 mm and 1 mm. Said thickness can preferably be between 50 µm and 600 µm, more preferred between 100 µm and 500 µm, and even more preferred between 200 µm and 500 µm In certain embodiments the thickness of the sheath is 50 µm, 100 µm, 200 µm, 300 µm, 400 µm, 500 µm, or 600 µm.

A person skilled in the art will in view of the present invention understand that a thin sheath can contain less active ingredient than a thicker sheath; and that the concentration of the progestational steroid in the sheath should be enough to sustain release over the desired treatment period. By using an ethylene-vinyl acetate copolymer grade with higher or lower vinyl acetate-content the sheath's thickness can be altered while maintaining essentially the same average release rate of the estrogenic steroid. For instance, if a thicker sheath is desired because more of the progestational steroid has to be accommodated in the sheath, an ethylene-vinyl acetate copolymer grade with higher vinyl acetate content can be chosen.

In the context of the present invention the term intravaginal ring, also contemplates ring designs or structures, which have other shapes, e.g. polygonal shapes and/or wavy shapes, or where the structure is not a complete and/or closed circle/shape.

In a first preferred embodiment the three-layered intravaginal ring comprises
- a inactive core, i.e. a core without any active properties
- an intermediate layer comprising a first ethylene-vinyl acetate copolymer having a vinyl acetate content from 28 wt% to 33 wt%, and at least 5 wt% of an estrogenic steroid preferably at least 10 wt% of an estrogenic steroid, based on the weight of the intermediate layer, and wherein said estrogenic steroid are incorporated into the first ethylene-vinyl acetate copolymer in the form of particles, and
- a outer sheath comprising a second ethylene-vinyl acetate copolymer having a vinyl acetate content of 24 wt% to 40 wt%; and between 25 and 35 wt%, e.g. 27 or 33.9 wt% of an progestational steroid based on the weight of the sheath, and wherein said progestational steroid are incorporated into the second ethylene-vinyl acetate copolymer in the form of particles.

Said embodiment has a cross-sectional diameter of 5 mm, the intermediate layer a thickness of 50 - 300 µm and the sheath a thickness of 400 µm.

In a second preferred embodiment the three-layered intravaginal ring comprises
- a core comprising a first ethylene-vinyl acetate copolymer having a vinyl acetate content from 28 wt% to 33 wt%, and at least 5wt%of an estrogenic steroid, preferably at least 10 wt% of an estrogenic steroid based on the weight of the core, and wherein said estrogenic steroid are incorporated into the first ethylene-vinyl acetate copolymer in the form of particles,
- a inactive intermediate layer i.e. an intermediate layer without any active properties, and
- a outer sheath comprising a second ethylene-vinyl acetate copolymer having a vinyl acetate content of 24 wt% to 40 wt%, and between 25 and 35 wt%, e.g. 27 or 33.9 wt% of an progestational steroid based on the weight of the sheath, wherein said progestational steroid are incorporated into the second ethylene-vinyl acetate copolymer in the form of particles.

Said embodiment has a cross-sectional diameter of 5 mm, the intermediate layer a thickness of 50 - 200 µm and the sheath a thickness of 400 µm.

In a preferred embodiment the average release rate of estradiol from a three-layered intravaginal ring according to the invention is from 60 µg/day to 160 µg/day during a treatment period of at least 28 days, e.g. one month, and preferably a near zero order release rate is provided. However said average release rate may be higher e.g. up to 300 µg/day, e.g. up to about 250 µg/day, e.g. 180 µg/day or about 165 µg/day, and/or lower if desired.

Additionally or alternatively, the average release rate of progestogen may be from 3 mg/day to 10 mg/day, such as about 3 mg/day or 4 mg/day for a treatment period of at least 28 days, e.g. about a month. It is expected that the release rate of progestogen will decrease during the treatment period as the content of progestogen in the sheath is depleted.

People skilled in the art will based on the present application understand that combinations of the average release rates of estradiol and estradiol is contemplated within the scope of the present inventing. For instance, in one intravaginal ring the average release rate of estradiol is 80 µg/day and the average release rate of progestogen is 10 mg/day, in a different embodiment the average release rate of estradiol is 100 µg/day and the average release rate of progestogen is 5 mg/day, in a third embodiment the average release rate of estradiol is 160 µg/day and the average release rate of progestogen is 8 mg/day, etc.

In a preferred embodiment according to the present invention the intravaginal ring does not comprise more than two steroids, i.e. an estrogenic steroid and a progestational steroid, and/or does not comprise further cores and/or layers such as sheaths and membranes, i.e. the intravaginal ring according to the invention consists of a single core, a single intermediate layer completely surrounding said core and a single sheath completely surrounding said intermediate layer, and wherein the estrogenic steroid is placed either in the core or the intermediate layer, the a progestational steroid in the sheath, and wherein the core or intermediate layer that is not containing the estrogenic steroid, is an inactive layer.

The present invention also relates to a method of manufacturing the intravaginal ring according to the present invention.

Said method comprises
a. providing an inactive core,
b. providing an intermediate layer comprising a first ethylene-vinyl acetate copolymer having a vinyl acetate content from 26 to 40 wt%, and at least 5wt% estrogenic steroid based on the weight of the intermediate layer, and wherein said estrogenic steroid is incorporated into the second ethylene-vinyl acetate copolymer in the form of particles,
c. providing a sheath comprising a second ethylene-vinyl acetate copolymer having a vinyl acetate content from 20 to 40 wt%, and at least 10 wt% of an progestational steroid based on the weight of the sheath, said progestational steroid is incorporated into the first ethylene-vinyl acetate copolymer in the form of particles
d. co-extruding the core, intermediate layer and sheath into a fiber,
e. cutting the fiber into an appropriate length thereby providing a fiber element, and
f. combining the ends of fiber element to form the intravaginal ring.

In a modified method according to the invention, step a and b are respectively modified into a' and b' as follows:
a'. providing an core comprising a first ethylene-vinyl acetate copolymer having a vinyl acetate content from 26 to 40 wt%, and at least 5wt% estrogenic steroid based on the weight of the core, and wherein said estrogenic steroid is incorporated into the first ethylene-vinyl acetate copolymer in the form of particles, and
b'. providing an inactive intermediate layer.

Step c - f are the same in the two method according to the invention.

As the core, intermediate layer and sheath are co-extruded a very simple and inexpensive embodiment according to the invention is provided, however the core, intermediate layer and/or sheath can be formed in separate injection moulding or extrusion steps if preferred. Injection moulding and extrusion are well known in the art and will not be discussed further in this application.

During extrusion the two steroids will to some degree dissolve in the polymer melt as result of higher solubility at higher temperatures. Upon cooling the amount dissolved in access relative to the amount that can dissolve at room temperature should re-crystallize. If this process is delayed the result is a physically instable product.

It is accordingly preferred that the method further comprises a cooling step in which the provided fiber or fiber element is cooled to a temperature of 20°C or below for providing the crystals of the steroids in the sheath and the core or intermediate layer. This may e.g. be obtained by placing the fiber into a cooling water bath.

Without being bound by theory, the inventors believe that active substances dissolved at elevated extrusion temperatures in the polymer melts will re-crystallize in the sheath and the core/intermediate layer upon cooling as a result of a sharp drop of the saturation solubility of the active ingredients in the polymers. This process should preferably take place promptly to avoid severe super saturation which is undesirable. The re-crystallization process is believed to be facilitated by "seed" crystals and the relatively high concentration of active ingredient in the sheath and the core/intermediate layer, will result in a higher concentration of "seed" crystals, upon which recrystallization can occur when the fiber is cooled after coextrusion. Thus, in the cooling step prompt re-crystallization is facilitated by crystals surviving the extrusion process and for this reason a high drug load is desired in order to avoid that most or all crystals disappear during extrusion and no seeds are left to prompt re-crystallization.

It is preferred that said cooling step is performed immediately after step d., i.e. as fast as is practically possible from a production point of view, i.e. preferably within less than 30 minutes from completion of the fiber in step d, and even more preferred within a period of a few (1 - 5) seconds to 10 minutes from completion of the fiber in step d, and even more preferred not more than about 2 minutes from completion of said fiber.

It is preferred that the rings are produced by heat welding the fiber ends together without the addition of further EVA material or adhesive.

The invention will be explained in greater detail below, describing exemplary embodiments of a three-layered intravaginal ring according to the invention, wherein
Fig. 1 shows a perspective view of a first embodiment of a three-layered intravaginal ring according to the invention,
Fig. 2 shows a perspective view of a second embodiment of a three-layered intravaginal ring according to the invention,
Fig. 3 shows simulated release rates of estradiol from rings AE1, AE2, and AE3 having an inactive core, and a 300 µm intermediate layer loaded with 10 wt% estradiol (5 mm fiber diameter), all three-layers of the rings are made of EVA28,
Fig. 4 shows simulated release rates of estradiol from rings AE4, AE5, and AE6 having an inactive core, and a 300 µm intermediate layer loaded with 5 wt% of estradiol (5 mm fiber diameter), all three-layers of the rings are made of EVA28,
Fig. 5 shows simulated estradiol release rates from rings BE1, BE2, BE3 having an inactive core, and an intermediate layer having different thicknesses, all three-layers of the rings are made of EVA28,
Fig. 6 shows simulated estradiol average daily release from ring BE1 having an inactive core and a 100 µm e intermediate layer, all three-layers of the ring are made of EVA28,
Fig. 7 shows simulated estradiol average daily release from ring BE4 having an inactive core and a 100 µm intermediate layer, all three-layers of the ring are made of EVA28,
Fig. 8 shows simulated estradiol release rate from rings BE4, BE5, BE6 having an inactive core and different thicknesses of the intermediate layer, all three-layers of the ring are made of EVA28,
Fig. 9 shows simulated estradiol average daily release from rings CE1, CE2, and CE3 each having an inactive intermediate layer. The core and sheath are made from EVA 28 and the inactive intermediate layer is made of EVA 15,
Fig. 10 shows simulated estradiol average daily release from ring CE1 having an inactive intermediate layer made of EVA 15, and
Fig. 11 shows progesterone release from two IVRs comprising 33.9 wt% progesterone in the sheath and with 0.39 wt % and 10 wt% in the ring.

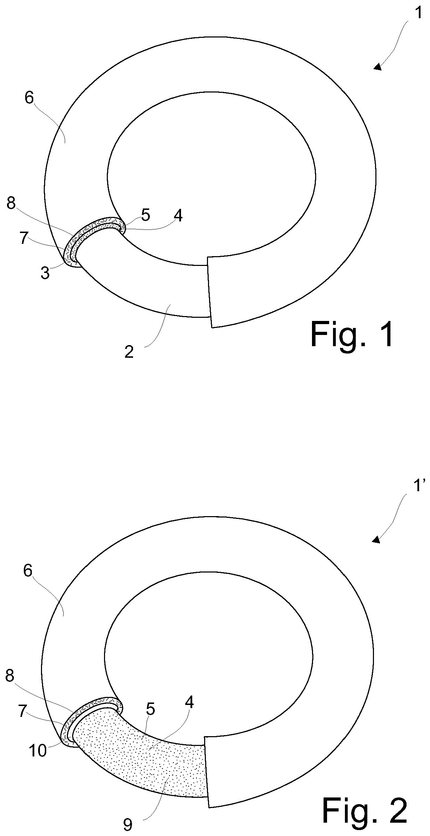

Figure 1 shows a preferred first embodiment of a three-layered intravaginal ring (IVR) 1 according to the invention. In said embodiment the IVR comprises an inactive core 2, an intermediate layer 3 made of a first ethylene-vinyl acetate copolymer 4 and an estrogenic steroid 5, and a sheath 6 made of a second ethylene-vinyl acetate copolymer 7 and comprising a progestational steroid 8. As is evident from the drawings, the three-layers of the intravaginal ring are co-axially arranged, i.e. the core, intermediate layer and sheath share a common axis.

Figure 2 shows a second embodiment of the three-layered intravaginal ring (IVR) 1' according to the invention. Said IVR corresponds to the first embodiment and for like parts the same reference numbers are used. In the second embodiment the core 9 is made of a first ethylene-vinyl acetate copolymer 4 and comprises the estrogenic steroid 5. and the intermediate layer 10 is the inactive layer. The sheath 6 is the same as for the first embodiment.

### Example 1: Effect of sheath thickness on the release of Estradiol (E2) from IVRs in which estradiol is present in the intermediate layer.

To evaluate the effect of the concentration of Estradiol in the intermediate layer and thickness of the sheath the release rate of a series of intra-vaginal rings having an inactive core has been evaluated using computational modeling. The model considers the diffusion of the Estradiol (E2) which is driven by the concentration gradient in the system and the dissolution of Estradiol crystals dispersed in the intermediate layer and it assumes the receiving medium to be a perfect sink. The release rate of Estradiol is affected by the Sheath thickness, and the concentration of Estradiol in the intermediate layer should be enough to provide a sustained release rate during 28 days of considered treatment.

Experimentally when rings are tested for *in-vitro* release, the medium is refreshed daily. Hence, the experimentally determined release rate is in fact the release rate that is averaged over 24 hours. For this reason, the simulated results have also been averaging over the period of one day.

The three layers of the tested vaginal rings are all made of EVA28, i.e. an ethylene-vinyl acetate copolymer having a vinyl acetate content of 28 wt%.

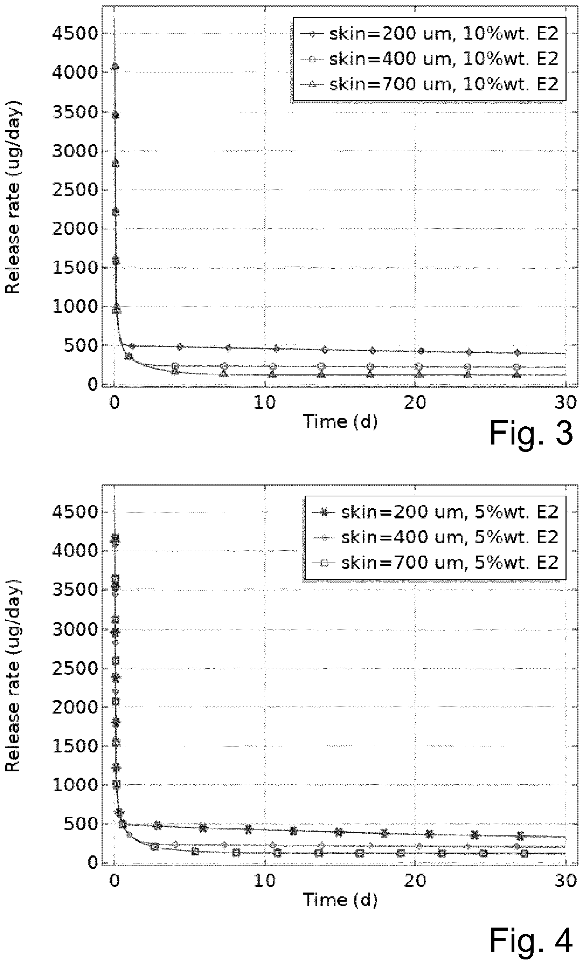

The average release rate of estradiol is provided in Table 1, and are shown in Fig. 3 and Fig. 4

**Table 1: Simulated release rates of Estradiol (E2)**

| | **Dimensions** | | | | **Drug loading** | | **Estradiol release** | | |
|---|---|---|---|---|---|---|---|---|---|
| Delivery system | Diameter (mm) | Length (mm) | Sheath (µm) | Intermediate layer EVA 28 (µm) | Intermediate layer wt% E2 EVA28 | Sheath wt% P4 | Burst (µg/day) | Average daily release (2-28) (µg/day) | Average daily release (1-28) (µg/day) |
| AE1 | 5 | 157 | 200 | 300 | 10.1 | 33.9 | 831.84 | 447.67 | 461.39 |
| AE2 | 5 | 157 | 400 | 300 | 10.07 | 33.9 | 782.50 | 234.12 | 253.70 |
| AE3 | 5 | 157 | 700 | 300 | 10.18 | 33.9 | 782.30 | 139.98 | 162.92 |
| AE4 | 5 | 157 | 200 | 300 | 5.00 | 33.9 | 829.92 | 402.26 | 417.53 |
| AE5 | 5 | 157 | 400 | 300 | 5.06 | 33.9 | 782.46 | 226.22 | 246.08 |
| AE6 | 5 | 157 | 700 | 300 | 5.18 | 33.9 | 782.27 | 138.75 | 161.73 |

The simulation shows that the average daily release from a ring having 33.9 wt% progesterone (PGN) crystals in the sheath act like a release rate-limiting membrane, i.e. it reduces the release rate of Estradiol from the ring. The presence of progesterone crystals increases the diffusion length and reduces the amount of available EVA.

It should be noted that the drug is homogeneously distributed after equilibration and no partitioning between core, intermediate layer and sheath will take place as all are made of EVA28.

### Example 2: Effect of the intermediate layer thickness and its Estradiol concentration on the release rate from IVRs in which Estradiol is present in the intermediate layer.

A parametric study is conducted to demonstrate the release rate regarding the thickness of the intermediate layer and its Estradiol concentration from an intravaginal ring comprising an inactive core.

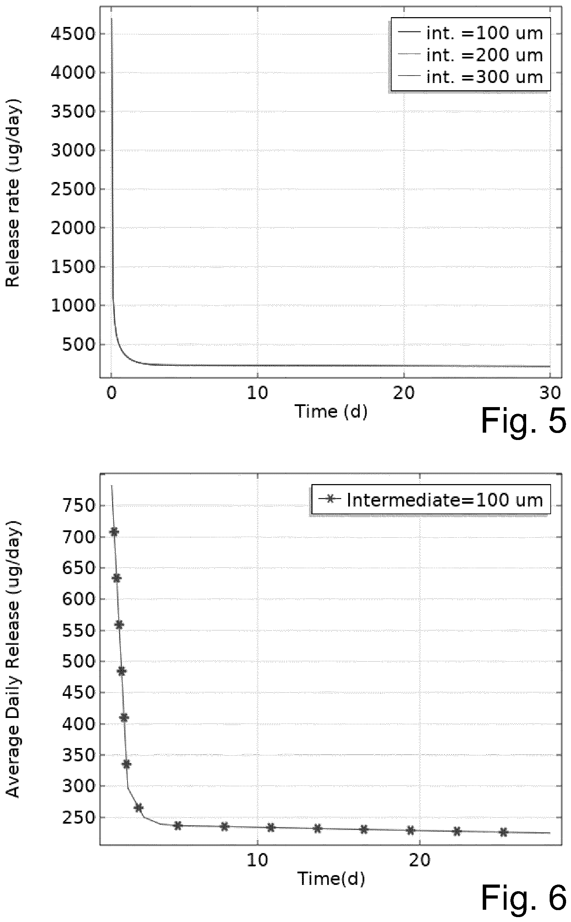

The results are shown in Table 2 and in Fig. 5 to Fig. 8.

**Table 2: Simulated release rates of Estradiol.**

| | **Dimensions** | | | | **Drug loading** | | **Estradiol release** | | |
|---|---|---|---|---|---|---|---|---|---|
| Delivery system | Diameter (mm) | Length (mm) | Sheath (µm) EVA28 | InterMediate layer (µm) EVA28 | Intermediate layer wt% E2 | Sheath wt% P4 | Burst (µg/day) | Average daily release (2-28) (µg/day) | Average daily release (1-28) (µg/day) |
| BE1 | 5 | 157 | 400 | 100 | 12.17 | 33.9 | 782.51 | 234.14 | 224.73 |
| BE2 | 5 | 157 | 400 | 200 | 10.60 | 33.9 | 782.50 | 234.14 | 224.73 |
| BE3 | 5 | 157 | 400 | 300 | 10.07 | 33.9 | 782.50 | 234.14 | 224.73 |
| BE4 | 5 | 157 | 400 | 100 | 6.28 | 33.9 | 782.44 | 220.80 | 187.53 |
| BE5 | 5 | 157 | 400 | 200 | 4.58 | 33.9 | 782.44 | 221.40 | 200.84 |
| BE6 | 5 | 157 | 400 | 300 | 4.04 | 33.9 | 782.44 | 221.41 | 200.85 |

Fig. 5 and 6 shows the release rate of Estradiol and the average daily release for different intermediate layer thicknesses and for an Estradiol concentration around 10 wt%.

Said results shows that for a concentration of about 10 wt% Estradiol, no visible impact can be observed on the release rate of Estradiol during the treatment period. The release rate is mainly governed by the Sheath thickness loaded with progesterone crystals, as dissolved Estradiol can be maintained at saturation during the treatment period (i.e., enough Estradiol is present in the intermediate layer even for the 100 µm) .

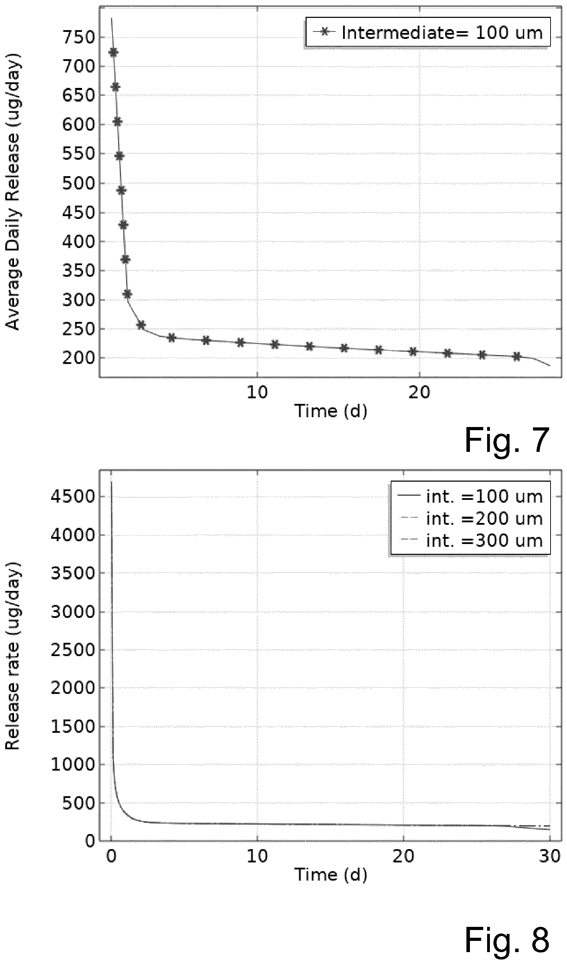

For a concentration of Estradiol around 5 wt% the release rate is comparable to 10 wt% Estradiol for all the rings, however for ring BE4 (intermediate 100 µm) a depletion can be seen around day 27 as shown in Fig. 7 and Fig. 8.

### Example 3: Effect on release rate of estradiol present in the core and the thickness of an inactive intermediate layer.

To evaluate the effect of the thickness of an inactive intermediate layer, different intra-vaginal rings have been evaluated using computational modeling.

Increasing the sheath thickness to reduce the release of Estradiol could be used to a certain level to tailor the release rate of Estradiol. However, achieving a low release of Estradiol e.g. 80 µg/day may lead to a very thick sheath which might require longer maturation time for the equilibration of Estradiol in the intravaginal rings.

In this example the IVRs comprise Estradiol in the core (of EVA 28), a rate-limiting intermediate layer made of EVA 15, and a sheath made of EVA2 8 and comprising 33.9wt% progesterone.

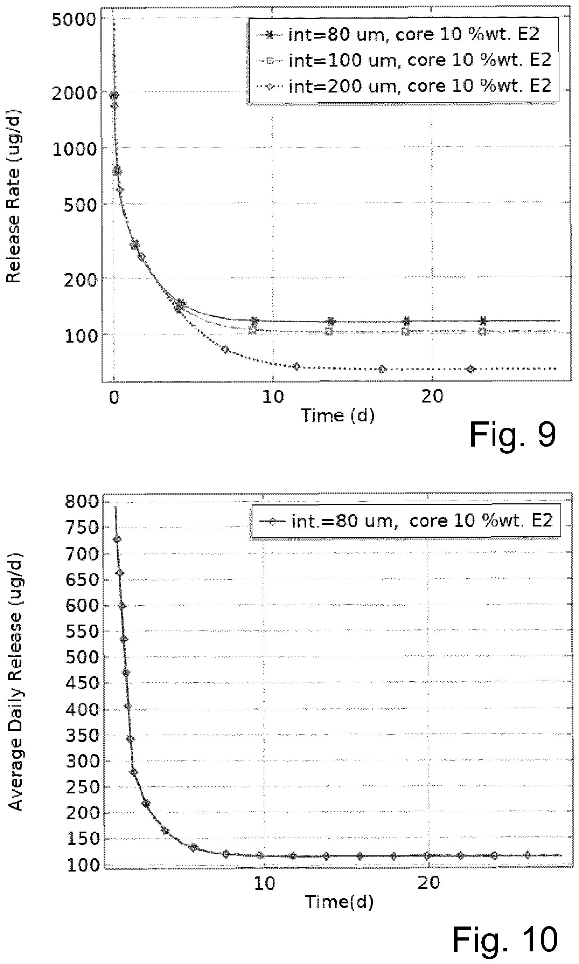

The results of the release rate for estradiol is provided in Table 3 and shown in Fig. 9 and Fig. 10.

**Table 3: Simulated release rates of estradiol for IVRs having an core made of EVA28, an inactive intermediate layer made of EVA15, and a sheath made of EVA28 .**

| | **Dimensions** | | | | **Drug loading** | | **Estradiol release** | | | |
|---|---|---|---|---|---|---|---|---|---|---|
| Delivery system | Diameter (mm) | Length (mm) | Sheath (µm) EVA28 | InterMediate layer (µm) EVA15 | Core wt% E2 | Sheath wt% P4 | Burst (µg/day) | Average daily release (2-28) (µg/day) | Average daily release (1-28) (µg/day) | Day 28 release (µg/day) |
| CE1 | 5 | 157 | 400 | 80 | 10 | 33.9 | 791.36 | 128.64 | 152.31 | 115.15 |
| CE2 | 5 | 157 | 400 | 100 | 10 | 33.9 | 791.36 | 117.17 | 141.24 | 101.75 |
| CE3 | 5 | 157 | 400 | 200 | 10 | 33.9 | 791.36 | 85.994 | 111.19 | 63.606 |

As is evident from said results incorporation of an inactive intermediate layer of EVA 15, lead to a substantial reduction in the release rate of Estradiol. The reduction is dependent also on the thickness of the intermediate layer; thus the release rate can be tailored to meet a desired release rate of Estradiol, even for an average release rate lower than 80 µg/day, independently of the release of progesterone from the sheath.

### Example 4: Release of progesterone

In order to compare the release rate of progesterone in the dependence of concentration of estradiol in an intravaginal ring, the following IVRs were tested:
AC400: An IVR with a sheath made of EVA28 and comprising 33.9 wt% progesterone, said ring contains 0.39 wt% estradiol in a layer made of EVA28. Cross-sectional fiber diameter: 5.00 mm.
DC400: An IVR with a sheath made of EVA28 and comprising 33.9 wt% progesterone, said ring contains 10 wt% estradiol in a layer made of EVA28. Cross-sectional fiber diameter: 5.00 mm.

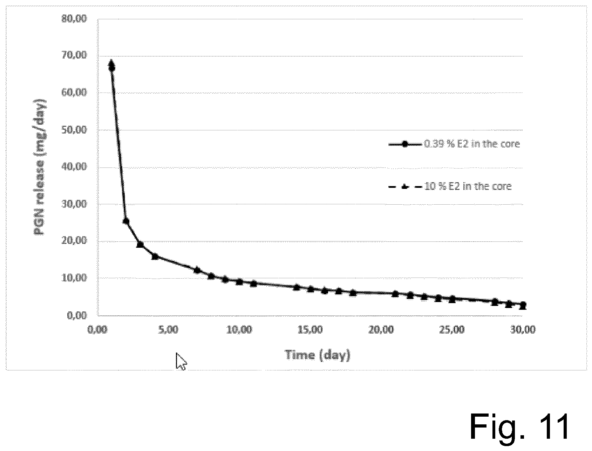

The progesterone release rates are shown in table 4 and Fig. 11

**Table 4: Mass of Progesterone released during each sampling interval (mg)**

| **Time (days):** | **0-6** h | **6-24** h | **1,00** | **2,00** | **3,00** | **4,00** | **7,00** | **8,00** | **9,00** | **10,00** | **11,00** | **14,00** |
|---|---|---|---|---|---|---|---|---|---|---|---|---|
| **AC400 Mean (mg)** | 34,84 | 32,02 | 66,85 | 25,66 | 19,22 | 16,02 | 12,38 | 10,79 | 9,82 | 9,19 | 8,64 | 7,71 |
| **DC400 Mean (mg)** | 35,84 | 32,49 | 68,32 | 25,88 | 19,32 | 16,09 | 12,56 | 10,79 | 9,96 | 9,29 | 8,67 | 7,82 |
| | | | | | | | | | | | | |

| **Time (days):** | **15,00** | **16,00** | **17,00** | **18,00** | **21,00** | **22,00** | **23,00** | **24,00** | **25,00** | **28,00** | **29,00** | **30,00** |
|---|---|---|---|---|---|---|---|---|---|---|---|---|
| **AC400 Mean (mg)** | 7,24 | 6,81 | 6,61 | 6,28 | 5,87 | 5,63 | 5,26 | 4,94 | 4,66 | 4,01 | 3,56 | 3,22 |
| **DC400 Mean (mg)** | 7,33 | 6,92 | 6,66 | 6,31 | 5,92 | 5,55 | 5,23 | 4,81 | 4,45 | 3,70 | 3,16 | 2,79 |

It can be seen from said results that the concentration of estradiol in the ring does not affect the release of progesterone, as both IVRs AC400 (0.39 wt% estradiol) and DC400 (10 wt% estradiol) have substantially identical release profiles of progesterone. Thus, it can be concluded from said data that the release rate of progesterone is independent of the concentration of estradiol in the ring,

Using the intravaginal ring according to the present invention the inventors have found that it is possible to attain independent and optimal release of the two active ingredients; an estrogenic steroid and a progestational steroid without the need for complex assembly of parts and without the need to use sophisticated multi-layer extrusion technology.

The intravaginal ring has a simple and inexpensive design, and can therefore be used equally well both privately and in medical or hospital facilities.

Modifications and combinations of the above principles and designs are foreseen within the scope of the present invention.

## Claims

1. An three-layered intravaginal ring (1;1') comprising
- a core (2;9)
- an intermediate layer (3;10), and
- a sheath (6) comprising a second ethylene-vinyl acetate copolymer (7) having a vinyl acetate content from 20 to 40 wt%, and at least 10 wt% of a progestational steroid (8) based on the weight of the sheath, wherein
one of the core (9) and intermediate layer (3) comprises a first ethylene-vinyl acetate copolymer (4) having a vinyl acetate content from 26 to 40 wt% and an estrogenic steroid (5), and
the other one of the core (2) and intermediate layer (10) is an inactive layer (2;10).

2. A three-layered intravaginal ring according to claim 1, wherein the sheath (6) comprises at least 15 wt% of the progestational steroid (8) based on the weight of the sheath (6), preferably at least 20 wt%, and even more preferred at least 30 wt% and/or wherein the sheath comprises 40 wt% or less of the progestational steroid (8) based on the weight of the sheath, preferably 35 wt% or less.

3. A three-layered intravaginal ring according to claim 1 or 2, wherein the progestational steroid (8) is dispersed and/or incorporated in the second ethylene-vinyl acetate copolymer (7) in the form of particles, such as crystals.

4. A three-layered intravaginal ring according to according to any of the preceding claims, wherein the estrogenic steroid (5) is dispersed and/or incorporated in the first ethylene-vinyl acetate copolymer (4) in the form of particles, e.g. crystals.

5. A three-layered intravaginal ring according to according to any of the preceding claims, wherein the core (9) or intermediate layer (3) comprises at least 5 wt% of the estrogenic steroid (5) based on the weight of the core or intermediate layer, preferably at least 10 wt%, and even more preferred at least 15 wt%.

6. A three-layered intravaginal ring according to any of the claims 3, 4 or 5 wherein the particles of the progestational steroid (8) and the estrogenic steroid in the second and the first ethylene-vinyl acetate copolymer (7,4), have a particle size of between 1 µm and 40 µm, preferably between 2 µm and 24 µm, and even more preferred between 3 and 10 µm, and even more preferred around 5µm as determined by laser diffraction.

7. A three-layered intravaginal ring according to any of the preceding claims, wherein the estrogenic steroid (5) is estradiol or estriol.

8. A three-layered intravaginal ring according to any of the preceding claims, wherein the progestational steroid (8) is selected from a group consisting of progestogen, progesterone, etonogestrel, levonorgestrel, d-1-norestrel, segesterone and norethindrone.

9. A three-layered intravaginal ring according to any of the preceding claims, wherein said intravaginal ring only comprises one estrogenic steroid (5) and one progestational steroid (8).

10. An intravaginal ring according to any of the preceding claims, wherein said intravaginal ring only comprises a single core, a single intermediate layer completely surrounding said core and a single outer sheath completely surrounding said intermediate layer.

11. A three-layered intravaginal ring according to any of the preceding claims, wherein when the intermediate layer (3) comprises the estrogenic steroid (5), said intermediate layer (3) preferably has a thickness from 50 µm to 500 µm, preferably from 80 µm to 400 µm, and even more preferred from 100 µm to 300 µm.

12. A three-layered intravaginal ring according to any of the preceding claims 1 - 10, wherein when the intermediate layer (10) is the inactive layer, said intermediate layer (10) preferably has a thickness from 50 µm to 300 µm, preferably from 50 µm to 200 µm, and even more preferred from 80 µm to 200 µm.

13. A three-layered intravaginal ring according to any of the preceding claims, wherein the cross-sectional diameter of the core (2;9) is from between 2 and 8 mm, more preferably between 3 mm and 6 mm and even more preferably from 4 mm to 5 mm.

14. A three-layered intravaginal ring according to any of the preceding claims, wherein the thickness of the sheath (6) is between 0.05 mm and 1 mm, preferably between 50 µm and 600 µm, more preferred between 100 µm and 500 µm, and even more preferred between 200 µm and 500 µm.

15. A method of manufacturing the three-layered intravaginal ring (1) according to any of the claims 1 - 14, said method comprises
a. providing an inactive core (2),
b. providing an intermediate layer (3) comprising a first ethylene-vinyl acetate copolymer (4) having a vinyl acetate content from 26 to 40 wt%, and at least 5wt%, preferably at least 10 wt% estrogenic steroid (5) based on the weight of the intermediate layer, and wherein said estrogenic steroid is incorporated into the first ethylene-vinyl acetate copolymer (4) in the form of particles,
c. providing a sheath (6) comprising a second ethylene-vinyl acetate copolymer (7) having a vinyl acetate content from 20 to 40 wt%, and at least 10 wt% of an progestational steroid (8) based on the weight of the sheath, wherein said progestational steroid is incorporated into the second ethylene-vinyl acetate copolymer (4) in the form of particles,
d. co-extruding the core (2;9), intermediate layer (3;10) and sheath (6) into a fiber, and
e. cutting the fiber into an appropriate length thereby providing a fiber element, and
f. combining the ends of fiber element to form the three-layered intravaginal ring.

16. A method according to claim 15, wherein step a and b of the method of claim 15 are modified as a' and b' respectively:
a'. providing an core (9) comprising a first ethylene-vinyl acetate copolymer (4) having a vinyl acetate content from 26 to 40 wt%, and at least 5wt%, preferably at least 10 wt% estrogenic steroid (5) based on the weight of the core, and wherein said estrogenic steroid is incorporated into the first ethylene-vinyl acetate copolymer in the form of particles, and
b'. providing an inactive intermediate layer (10).

17. A method according to claim 15 or 16, wherein said method further comprises a cooling step in which the provided fiber from step d. is cooled to a temperature of about 20°C in order to provide crystals of the progestational steroid (8) and the estrogenic steroid (5).

18. A method according to claim 17, wherein said cooling step is completed within a periode of less than 10 minuts from completion of the fiber in step d, and even more preferred not more than about 2 minutes from completion of the fiber in step d.
